# EUROPEAN PATENT SPECIFICATION

(11) **EP 2 249 767 B1**
(45) Date of publication and mention of the grant of the patent: **10.08.2016**
(21) Application number: 09719501.0
(22) Date of filing: 06.03.2009
(51) Int. Cl.: A61K 8/81, A61K 8/86, A61K 8/37, A61K 8/44, A61K 8/60, A61K 8/04, A61Q 1/14

(54) **Make-up removal composition which provides good foam**
Zusammensetzung mit gutem Schaumvermögen zur Entfernung von Makeup
Composition démaquillante à excellent pouvoir moussant

(30) Priority: 12.03.2008 GB 0804479
(43) Date of publication of application: 17.11.2010
(73) Proprietor: Unilever PLC, a company registered in England and Wales under company no. 41424, London EC4Y 0DY (GB); Unilever N.V., 3013 AL Rotterdam (NL)
(72) Inventor: HATTORI, Shizuma, Tochigi 321-3325 (JP); MORIMOTO, Shota, Tochigi 321-3325 (JP); MURATA, Yukihiro, Tochigi 321-3325 (JP); NUMATA, Tadashi, Tochigi 321-3325 (JP)
(74) Representative: Fijnvandraat, Arnoldus
(86) International application number: PCT/EP2009/052653
(87) International publication number: WO 2009/112432

(56) References cited:
- EP-A- 0 687 721
- EP-A- 1 433 476
- EP-A1- 0 648 833
- WO-A-00/72807
- WO-A-2005/034895
- US-A- 5 977 037
- US-A1- 2005 180 939

## Description

### FIELD OF THE INVENTION

The present invention relates to makeup removal compositions which also provide good foam,

### BACKGROUND OF THE INVENTION

Make-up remover is typically applied to skin as a liquid oil composition and has little or no foam. It would be advantageous to find a make-up composition which uses less oil and rinses off more easily, but retains some make-up removing efficiency.

By utilizing specific foaming anionic surfactant, applicants can provide foaming make-up remover which uses less oil (e.g., hydrogenated polyisobutene) and rinses off more readily To ensure the efficiency of make-up removal and stability of such composition, a specific combination of amphoteric (hydroxysultaine), nonionic and C₁₀-C₁₄ ester oil are also needed. It has been found that certain levels of specific nonionics, including at least 0.1% alkyl glucoside, are required to maintain the overall efficiency and stability.

Three references which display general knowledge, but not the specific combinations of the invention are:
(1) JP 2005-200640 to NOF Corporation;
(2) JP H09-175936 to Kao; and
(3) JP H06-016524 to Kao

Additional references which display general knowledge but not the specific combinations of the invention are:
EP 0687 721 discloses a detergent composition containing an N-acylthreonine salt in combination with a higher fatty acid salt. The detergent composition exhibits little irritation and has improved foam maintenance, foam quality and feel upon use.
US 2005/180939 discloses a foaming skin cleansing composition comprising a physiologically acceptable medium, a surfactant system containing at least one anionic surfactant and at least one amphoteric surfactant. The composition also comprises a thickening system containing at least two different anionic polymers each comprising at least one hydrophobic chain.
EP-A-1433476 describes a stain cleansing composition comprising (a) an oil component, (b) a hydrophilic nonionic surfactant, (c) a lipophilic amphiphile, (d) a water soluble solvent and (e) water, the composition having an isotropic liquid phase exhibiting a bicontinuous structure. The composition is said to exhibit excellent detergency for the removal of both oil and water soluble stains, and has high rinsability.
EP-A-648833 describes a detergent composition comprising a salt (a) of an N-acyl derivative of an amino acid selected from glycine, clanine and β-alanine, and a salt (b) of a higher fatty acid in a weight ratio of (a):(b) of 99.5:0.5 to 90:10, wherein the acyl residue is a residue of a C₈-C₂₀ (un)saturated fatty acid; and another detergent composition which contains a higher alcohol and/or a polyhydric alcohol in addition to the above components. The invention is said to improve the performance of N-acylated amino acid salts as the detergent

### BRIEF SUMMARY OF THE INVENTION

More specifically, the present invention comprises a foaming, easier to rinse make-up removing composition comprising:
(1) 1 to 10%, preferably 1.5 to 5% by wt. salts of N-acyl amino acid, represented by the following formula:

   R'CONH(CH₂)ₙCOOM,

   wherein R'CO is straight chain acyl of C₈-C₂₀ carbons; n = 1 or 2 and M is alkali metal salt or trialkanolamine;
   e.g., N-acyl glycinate; especially preferred is C₁₀-C₁₆ alkali metal glycinate, e.g., acyl potassium glycinate;
(2) 1 to 10%, preferably 2 to 4% by wt. of an amphoteric surfactant comprising alkyl hydroxy sultaine;
(3) 0.1 to 10%, preferably 3 to 5% by wt. hydrogenated polyisobutene
(4) 0.1 to 10% by wt. nonionic surfactant comprising alkyl glucoside and alkoxylated glyceryl, except that there is at least 0.1, preferably at least 0.4% by wt. alkyl glucoside;
(5) 0.1 to 5%, preferably 1 to 3% by wt. C₁₀-C₁₄ ester, preferably laurate ester wherein preferably the ester (e.g., laurate ester) is free of hydroxyl functional group; and
(6) balance water.

Compositions of the invention are preferably dispensed in a manner that the volume is about 5-20 times greater when dispensed relative to prior to dispensing. This is preferably accomplished using, for example a pump foamer dispenser package. Such package is beneficial, for example, for applying composition onto the face without dripping.

These and other aspects, features and advantages will become apparent to those of ordinary skill in the art from a reading of the following detailed description and the appended claims. For the avoidance of doubt, any feature of one aspect of the present invention may be utilized in any other aspect of the invention. It is noted that the examples given in the description below are intended to clarify the invention and are not intended to limit the invention to those examples per se. Similarly, all percentages are weight/weight percentages of the total composition unless otherwise indicated. Numerical ranges expressed in the format "from x to y" are understood to include x and y. When for a specific feature multiple preferred ranges are described in the format "from x to y", it is understood that all ranges combining the different endpoints are also contemplated. Where the term "comprising" is used in the specification or claims, it is not intended to exclude any terms, steps or features not specifically recited. All temperatures are in degrees Celsius (°C) unless specified otherwise. All measurements are in SI units unless specified otherwise. All documents cited are - in relevant part - incorporated herein by reference.

### DETAILED DESCRIPTION OF THE INVENTION

The present invention provides compositions that achieve make-up removal efficiency (MUR efficiency), while also providing good foaming (which actually helps with make-up removal efficiency), while using less oil. Moreover, all this is done while maintaining good stability of the compositions. This is accomplished through specific selection of compounds which interact with one another to maintain the delicately defined balance.

More specifically, good make-up removal efficiency is obtained using combination of hydrogenated polyisobutene (oil) and alkyl hydroxy sultaine. Use of the hydroxysultaine permits the polyisobutene to be used at relatively low levels of 3 to 5% by wt. Less oil is also used because of use of salts of N-alkoyl amino acid (e.g., N-alkyl glycinate) as foaming anionic. Nevertheless, such systems are not stable unless there is also used, as a type of stabilization system, about 3 to 8% nonionic surfactant, where at least 1% of composition comprises alkyl glucoside, in combination with 1 to 3% by wt of a C₁₀ C₁₄ ester oil, preferably laurate.

The composition is described in greater detail below.

Compositions of the invention comprise 1 to 10%, preferably 1 5 to 5% by wt. salts of N-acyl amino acid represented by the following formula:

R'CONH(CH₂)ₙCOOM

wherein R'CO is a straight chain acyl of C₈ to C₂₀, preferably C₁₀ to C₁₈ carbons n = 1 or 2, and
M is alkali metal (e.g., sodium, potassium) or trialkanolamine.

An especially preferred compound is C₁₀-C₁₆ alkali metal glycinates, e.g., acyl potassium glycinate.

The anionic is important because, in combination with hydroxysultaine, it allows less oil (e.g., polyisobutene) to be used while still retaining good foaming. Stability of the oil, as discussed below, is further ensured through use of non ionic and ester oil stabilization system.

A second required component of the subject invention is amphoteric surfactant wherein said amphoteric comprises alkyl hydroxysultaine (e.g., lauryl hydroxysultaine). It is believed that the amphoteric helps with cleanser efficiency while also helping with overall stability, principally provided by nonionic and ester combination.

The amphoteric is used in an amount of 1 to 10%, preferably 2 to 4% by wt. of the composition.

A third component of the compositions of the invention is hydrogenated polybutene The hydrogenated polybutene oil, typically a good make-up remover, can be used in amounts of 0.1 to 5%, preferably 3 to 5% by wt. Higher amounts are not needed because, as noted, efficacy as provided by good foaming (as provided by N-acyl amino acid and hydroxysultaine) and combination of other ingredients. This permits also that the compositions have less greasy/oily feel than might be otherwise expected (e.g., according to consumer in-use testing).

Although less oil is used, stability is further provided through a combination of nonionic surfactant (which must comprise alkyl glucoside and alkoxylated glyceryl, except that there must be at least 0.1, preferably at least 0.4% alkyl glucoside); and C₁₀-C₁₄ ester. The amphoteric also helps with stability

Specifically, the compositions comprise 0.1 to 10% nonionic surfactant comprising alkyl glucoside and alkoxylated glyceryl except that at least 0.1% wt. alkyl glycoside must be present.

In addition, the composition must comprise 0.1 to 5%, preferably 1 to 3% by wt. C₁₀-C₁₄ ester, preferably a laurate ester. Preferably the ester (e.g., laurate ester) is free of hydroxyl functional groups.

### The balance of the composition comprises water

Compositions of the invention also may comprise other ingredients that may be used in make-up remover compositions These include oils (e.g., ester oils, mineral oils and plant oils), silicone, fatty acids, alcohols, polyols, colorants, preservatives, perfumes, powders, scrubs, chelating agents, gelling agents and other ingredients such as may be well known to those skilled in the art of make-up removal formulations.

### EXAMPLES

### Protocol

Stability of compositions was measured by observing phase stability at ambient temperature (e.g., 20-25°C) after 24 hours. Compositions which did not phase separate were considered stable.

Make-up removal efficiency was defined by placing a dab of lipstick (about 0.008 g) on the arm (e.g., preferably the forearm). This is followed by applying the formula from a pump foamed package using a single push from the dispenser (dispensing about 12 cm³ formulation) and rubbing for about 60 seconds. The skin is then rinsed for about 30 seconds in water and a 5 point scale of visual assessment was used to determine removal.

Foam is measured by observing amount of foam released from pump foamer and grading based on 5 point visual scale.

### Examples

The table below with Examples 1 and Comparatives A-M show how each element of the invention is needed to provide ideal combination of MUR efficiency, foam, and phase stability. When some parameter or other is not met, there is typically some issue as noted in the description of the Comparatives.

**Table 1**

| Ingredients | Ex. 1 % by wt. | A | B | C | D | E | F | G | H | I | J | K | L | M |
|---|---|---|---|---|---|---|---|---|---|---|---|---|---|---|
| | | | | | | | | | | | | | | |
| Water | 68.4 | 72.9 | 71.1 | 69.9 | 68.8 | 71.9 | 71.4 | 69.75 | 72.9 | 69.9 | 73.3 | 83.4 | 68.4 | 68.4 |
| Cocoyl glyanate | 3 | 3 | 3 | 3 | 3 | 3 | - | 3 | 3 | 3 | 3 | 3 | 3 | 3 |
| Lauryl hydroxysultaine | 2.7 | 2.7 | - | 2.7 | 2.7 | 2.7 | 2.7 | 1.35 | 2.7 | 2.7 | 2.7 | 2.7 | Δ | 2.7 |
| Lauryl glucoside | 0.4 | 0.4 | 0.4 | 0.4 | 0.4 | 0.4 | 0.4 | 0.4 | 0.4 | 0.4 | - | 0.4 | 0.4 | 0.4 |
| PEG-20 glyceryl | 4.5 | 4.5 | 4.5 | 4.5 | 4.5 | 4.5 | 4.5 | 4.5 | - | 4.5 | - | 4.5 | 4.5 | 4.5 |
| Glycerin | 4.5 | 4.5 | 4.5 | 4.5 | 4.5 | 4.5 | 4.5 | 4.5 | 4.5 | 4.5 | 4.5 | - | 4.5 | 4.5 |
| Isopentyldiol | 5 | 5 | 5 | 5 | 5 | 5 | 5 | 5 | 5 | 5 | 5 | - | 5 | 5 |
| PEG-400 | 5.5 | 5.5 | 5.5 | 5.5 | 5.5 | 5.5 | 5.5 | 5.5 | 5.5 | 5.5 | 5.5 | - | 5.5 | 5.5 |
| Laurate ester | 1.5 | 1.5 | 1.5 | - | 1.5 | 1.5 | 1.5 | 1.5 | 1.5 | - | 1.5 | 1.5 | 1.5 | ** |
| Hydrogenated polyisobutene | 4.5 | - | 4.5 | 4.5 | 4.5 | 1 | 4.5 | 4.5 | 4.5 | 4.5 | 4.5 | 4.5 | 4.5 | |
| Stability | OK | OK | OK | Sep* | Sep* | OK | OK | OK | Sep* | Sep* | Sep* | OK | OK | Sep* |
| Appearance | T | T | O | | | T | S | O | | | | S | S | |
| MUR efficiency | 5 | | 1 | | | 4 | 3 | 2 | | | | 2 | 3 | |
| Foam quickly | 5 | No foam | 5 | | | 5 | 2 | 5 | | | | 5 | 5 | |

| | | | | | | | | | | | | | | |
|---|---|---|---|---|---|---|---|---|---|---|---|---|---|---|
| * Separates (i.e., phase separation) Δ Betaine used as amphoteric at 2.7% by wt. ** Isotridecyl isononanoate was used as oil at 1.5% by wt. T = Transparent O = Opaque S = Semitransparent | | | | | | | | | | | | | | |

What Comparatives Demonstrate.
- A: Shows you need oil (for foam formation),
- B: Shows you need hydroxysultaine for MUR efficiency,
- C: Shows you need ester for phase stability,
- D: Shows you also need nonionic (lauryl glucoside) for stability,
- E: Similar to Example 1, but with only 1% polybutene, there is less optimal MUR efficiency,
- F: With no salt of N Acyl amino acid, there is big drop in both MUR efficiency and foam;
- G: Shows that, without minimal sultaine, MUR efficiency is compromised;
- H: Shows that, w/o minimum nonionic, there is phase instability;
- I: Shows that ester oil is also required for stability,
- J: Again shows minimum nonionic needed for stability;
- K: Suggests that a certain amount of polyol is required for optimal MUR efficiencies;
- L: With different amphoteric, less MUR efficiency;
- M: With different oil, you get separation.

## Claims

1. A foaming, make-up remover composition comprising:
(a) 1-10% by wt. salts of N-acyl amino acid represented by the following formula:
R'CONH(CH₂)ₙCOOM,
wherein R'CO is straight chain acyl of C₈-C₂₀ carbons; n = 1 or 2 and M is alkali metal or trialkanolamine;
(b) 1-10% by wt. of an amphoteric surfactant comprising alkyl hydroxy sultaine;
(c) 0.1. to 10% by wt. hydrogenated polyisobutene;
(d) 0.1-10% nonionic surfactant comprising alkyl glucoside and alkoxylated glyceryl except that there must be at least 0.1 % alkyl glucoside;
(e) 0.1 to 5% by wt C₁₀-C₁₄ ester; and
(f) balance water.

2. A composition according to claim 1, wherein R'CO is straight chain acyl of C₁₀-C₁₈ carbons.

3. A composition according to claim 1 or claim 2, comprising to 2-4% by wt. hydroxy sultaine.

4. A composition according to any one of the preceding claims, wherein the C₁₀-C₁₄ ester is free of hydroxyl functional group.

## Patentansprüche

1. Schäumende Make-up-Entfernerzusammensetzung, umfassend:
(a) 1-10 Gew.-% Salze von N-Acylaminosäure, dargestellt durch die folgende Formel:
R'CONH(CH₂)ₙCOOM,
worin R'CO geradkettiges Acyl mit C₈-C₂₀-Kohlenstoffatomen bedeutet,
n = 1 oder 2 und
M Alkalimetall oder Trialkanolamin bedeutet,
(b) 1-10 Gew.-% eines amphoteren Tensids, umfassend Alkylhydroxysultain,
(c) 0,1 bis 10 Gew.-% hydriertes Polyisobuten,
(d) 0,1-10% nichtionisches Tensid, umfassend Alkylglucosid und alkoxyliertes Glyceryl, mit der Ausnahme, dass mindestens 0,1% Alkylglucosid vorliegen müssen,
(e) 0,1 bis 5 Gew.-% C₁₀-C₁₄-Ester und
(f) Rest Wasser.

2. Zusammensetzung nach Anspruch 1, wobei R'CO geradkettiges Acyl mit C₁₀-C₁₈-Kohlenstoffatomen bedeutet.

3. Zusammensetzung nach Anspruch 1 oder 2, umfassend 2-4 Gew.-% Hydroxysultain.

4. Zusammensetzung nach irgendeinem der vorhergehenden Ansprüche, wobei der C₁₀-C₁₄-Ester frei von hydroxylfunktioneller Gruppe ist.

## Revendications

1. Composition d'élimination de maquillage, moussante comprenant :
(a) 1-10 % en masse de sels d'acide N-acylaminé représentés par la formule suivante :
R'CONH(CH₂)ₙCOOM,
où R'CO est un acyle à chaîne linéaire de carbones en C₈-C₂₀ ; n = 1 ou 2 et M est un métal alcalin ou une trialcanolamine ;
(b) 1-10 % en masse d'un tensioactif amphotère comprenant une alkylhydroxysultaïne ;
(c) 0,1 à 10 % en masse de polyisobutène hydrogéné ;
(d) 0,1-10 % de tensioactif non ionique comprenant un alkylglucoside et un glycéryle alcoxylé à l'exception qu'il doit être d'au moins 0,1 % d'alkylglucoside ;
(e) 0,1 à 5 % en masse d'ester en C₁₀-C₁₄ ; et
(f) de l'eau pour le reste.

2. Composition selon la revendication 1, dans laquelle R'CO est un acyle à chaîne linéaire de carbones en C₁₀-C₁₈.

3. Composition selon la revendication 1 ou la revendication 2, comprenant 2-4 % en masse d'hydroxysultaïne.

4. Composition selon l'une quelconque des revendications précédentes, dans laquelle l'ester en C₁₀-C₁₄ est exempt de groupe hydroxyle fonctionnel.
